# Europäisches Patentamt
## European Patent Office
## Office européen des brevets

(19)

(11) Veröffentlichungsnummer: **0 374 686 B1**

(12) **EUROPÄISCHE PATENTSCHRIFT**

(45) Veröffentlichungstag der Patentschrift: **11.08.93**

(51) Int. Cl.⁵: **G01N 33/53**, G01N 33/569

(21) Anmeldenummer: **89122885.0**

(22) Anmeldetag: **12.12.89**

(54) **Verfahren zur Verbesserung von Richtigkeit und Reproduzierbarkeit der Messdaten immunometrischer Tests.**

(30) Priorität: **17.12.88 DE 3842580**

(43) Veröffentlichungstag der Anmeldung:
**27.06.90 Patentblatt 90/26**

(45) Bekanntmachung des Hinweises auf die Patenterteilung:
**11.08.93 Patentblatt 93/32**

(84) Benannte Vertragsstaaten:
**AT BE CH DE ES FR GB IT LI LU NL SE**

(56) Entgegenhaltungen:
**EP-A- 0 236 997**
**EP-A- 0 264 866**

**PATENT ABSTRACTS OF JAPAN, Band 12, Nr. 390 (P-772)[3237], 18. Oktober 1988; & JP-A-63 132 166 (OLYMPUS OPTICAL CO., LTD) 04-06-1988**

**PATENT ABSTRACTS OF JAPAN, Band 9. Nr. 329 (P-416)[2052], 24. Dezember 1985; & JP-A-60 154 161 (SHIMAZU SEISAKUSHO K.K.) 13-08-1985**

(73) Patentinhaber: **BEHRINGWERKE Aktiengesellschaft**
**Postfach 1140**
**W-3550 Marburg 1(DE)**

(72) Erfinder: **Dopatka, Hans-Detlef, Dr.**
**Heinrich Heine-Str. 9**
**W-3550 Marburg(DE)**
Erfinder: **Giesendorf, Bernhard, Dr.**
**Marktstr. 43**
**W-6255 Dornburg-Frickhofen(DE)**

(74) Vertreter: **Fischer, Hans-Jürgen, Dr. et al**
**Hoechst AG, Zentrale Patentabteilung, Gebäude F 821**
**W-6230 Frankturt am Main 80 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.6/3.3.1)

**Beschreibung**

Die Erfindung betrifft ein Verfahren zur Verbesserung von Richtigkeit und Reproduzierbarkeit der Meßdaten immunometrischer Tests, insbesondere solcher, die unter Verwendung von Mikrotitrationsplatten durchgeführt werden.

Wenn eine Untersuchungsprobe in der für den jeweiligen immunometrischen Test vorgeschriebenen Testverdünnung eingesetzt wird, liefert sie eine dem Test zugeordnete Meßzahl oder einen Meßwert. Ohne daß die Erfindung hierauf beschränkt werden soll, wird sie in ihren wesentlichen Grundzügen im folgenden anhand des Enzym-Linked-Immunosorbent-Assay (ELISA) als einem typischen Beispiel für die immunometrischen Tests beschrieben, auf die sie vorzugsweise angewendet werden kann. Bei dem ELISA wird die Messung über ein Photometer ausgewertet, wobei die Meßwerte Werte der optischen Dichte (o.D.) sind. Als Beispiel wird auch ein solcher Testaufbau angenommen, bei dem eine stärkere Analytkonzentration eine höhere o.D. liefert, d.h. das Beispiel ist kein kompetitiver Test. Aber auch dies stellt keine Einschränkung der Erfindung dar.

Soll der in einer Untersuchungsprobe im ELISA nachgewiesene Analyt quantifiziert werden, so kommen der Richtigkeit und der Reproduzierbarkeit des der Probe zuzuordnenden einzelnen Meßwertes entscheidende Bedeutung zu. Dabei ist es unerheblich, ob der Analyt beispielsweise ein Antigen oder ein Antikörper ist und wie die Quantifizierung erfolgt.

Da die Ursachen, die zu einer Verringerung der Richtigkeit und Reproduzierbarkeit von ELISA-Meßdaten führen, sehr vielfältig sind, ist eine grobe Klassifizierung hilfreich. Anhand dieser Einteilung kann dargelegt werden, welche Bemühungen bereits unternommen worden sind, um eine Verbesserung von Richtigkeit und Reproduzierbarkeit von immunometrischen Testmeßwerten, insbesondere ELISA-Meßwerten zu erreichen.

Es gibt keine idealen Bedingungen, um die Richtigkeit und Reproduzierbarkeit von ELISA-Tests zu bewerten. Es ist allgemein bekannt, daß jede Art von variablen Einflußgrößen wie Eigenart der Untersuchungsprobe, Reagenzienchargen, Konfektionierungsform der Reagenzien, technische Ausrüstung, Testdurchführender, Testzeitpunkt usw. das Meßergebnis mehr oder weniger beeinflußt. Diese Variablen können in gleicher Weise den Meßwert für die Untersuchungsproben wie für eine Kontrollprobe verfälschen.

Die Richtigkeit eines Meßwertes kann nur anhand einer Meßwertvorgabe überprüft werden. Dies wird in der Regel nicht bei den Untersuchungsproben, sondern bei der vom Reagenzienhersteller dem ELISA beigefügten Testkontrolle vorgenommen. Für das Ausgangsmaterial und die Sollwerterstellung dieser Testkontrolle gibt es genaue Vorschriften, bei denen durch geeignete Mittelwertbildung versucht wird, die Abweichungen vom Sollwert möglichst klein zu halten. Eine Bewertung des Sollwert-Ermittlungsmodelles des Verbandes der Diagnostika- und Diagnostikageräte-Hersteller e.V. ist von B. Müller-Wiegand et al, Lab. med. 10: 65-70 (1986), angegeben.

Bei der Reproduzierbarkeit eines Meßwertes wird zwischen der Präzision in der Serie (within-run precision) und der Präzision von Serie zu Serie (Between-run precision) unterschieden. Letzere beinhaltet z.B. die Präzision von Tag zu Tag oder von Labor zu Labor. Als Meßgröße sowohl für die Präzision in der Serie als auch für die von Serie zu Serie dient der Variationskoeffizient VK in %, wobei

$$VK = \frac{\text{Standardabweichung}}{\text{Mittelwert}} \times 100$$

ist. Detaillierte statistische Untersuchungen und Abschätzungen zur Reproduzierbarkeit wurden beispielsweise von M.J. Bookbinder und K.J. Panosian, Clin. chem. 32/9: 1734-1737 (1986), angegeben, wobei die Variation in der Serie mit der Variation von Serie zu Serie verglichen wird.

Die Reproduzierbarkeit innerhalb der Serie wird zu einem wesentlichen Anteil durch die Qualität der Reagenzien und die technische Ausrüstung bestimmt. W. Ehnert, Lab. med. 3: A + B 75-76 (1979), diskutiert Verbesserungen in der Bestimmung der Standardabweichungen in der Serie durch Mehrfachbestimmungen. Es ist bekannt, daß die Präzision durch eine Doppelbestimmung der Untersuchungsproben verbessert werden kann. Doppelbestimmungen der Untersuchungsproben erhöhen jedoch die Kosten stark und sind deshalb unwirtschaftlich.

2

Weiterhin ist eine Doppelbestimmung von Untersuchungsproben bei ELISA-Tests, die auf Mikrotitrationsplatten durchgeführt werden, dann von begrenztem Wert, wenn die zweite Bestimmung in benachbarter Positionierung vorgenommen wird, wie sich beispielsweise aus Untersuchungen von B.W. Stemshorn et al., J. of Immunol. meth., 61: 367-375 (1983), ergibt. Wirksamer ist es, die zweite Bestimmung in einer genau vorgeschriebenen, entfernten Anordnung zur Erstbestimmung in der Mikrotitrationsplatte vorzunehmen. Dieses Verfahren ist jedoch teuer und für den Testanwender kaum praktikabel.

Die Reproduzierbarkeit von Serie zu Serie wird durch eine Fülle von kaum definierbaren Faktoren beeinflußt. Üblicherweise wird zur Meßwertkorrektur der Untersuchungsproben auf die mitgeführte Testkontrolle Bezug genommen. Diese Testkontrolle liefert einen meistens als Mittelwert einer Mehrfachbestimmung bestimmten "Tageswert". Durch Vergleich dieses aktuellen Meßwertes mit dem Sollwert der Testkontrolle wird ein Korrekturfaktor ermittelt, anhand dessen die Meßwerte der Untersuchungsproben jeweils im gleichen Sinn korrigiert werden, wie es beispielsweise von A. Voller et al. in "The Enzyme Linked Immunosorbent Assay (ELISA)", A guide with Abstracts of Microplate Applications, 1979 beschrieben worden ist. Es wird also aufgrund des "Tageswertes" der Testkontrolle eine für den Tag geltende Abweichung des Sollwerts der Testkontrolle, d.h. ein Trend, angenommen, der zumindest teilweise als repräsentativ auch für die Meßwerte der Untersuchungsproben angesehen wird ("Niveau-Korrektur").

Die Korrektur für die Meßwerte der Untersuchungsproben wird so durchgeführt, daß die Meßwerte der Untersuchungsproben mit einem Faktor multipliziert werden, der gleich dem Faktor der Abweichung des Tageswertes der Testkontrolle vom Sollwert ist.

Es ist bekannt, daß bei ELISA-Tests, die in Mikrotitrationsplatten durchgeführt werden, die Reproduzierbarkeit von Serie zu Serie durch eine wechselnde Blockanordnung der Untersuchungsproben verbessert werden kann, wie von P.M. Burrows et al., J.Virol.Meth., 8:207-216 (1984) beschrieben ist. Dieses Verfahren stellt hohe Anforderungen an den Testdurchführenden, ist aufwendig und konnte sich in der Praxis aus Praktikabilitätsgründen nicht durchsetzen.

Bei Untersuchungen an ELISA-Tests auf Mikrotitrationsplatten zur Verbesserung der Interpretation der Meßwerte hat nun der Erfinder der vorliegenden Erfindung gefunden, daß sich innerhalb der Serie der Proben auf einer Mikrotitrationsplatte die Abweichung vom Sollwert verändert. Diese Abweichung besteht darin, daß sich gegenläufig zur Reihenfolge, mit der die Untersuchungsproben in die Mikrotitrationsplatte einpipettiert werden, eine Verfälschung der Meßwerte ergibt. Diese Verfälschung kann einfach demonstriert werden, indem eine Mikrotitrationsplatte ausschließlich mit der positiven Testkontrolle beschickt wird, die als Simulation der Sequenz der Untersuchungsproben interpretiert wird.

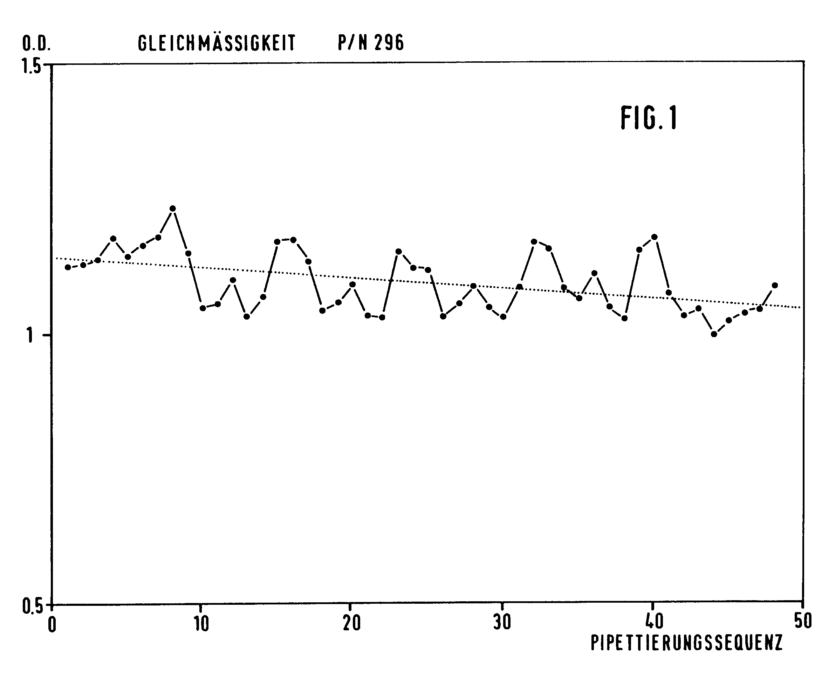

Figur 1 zeigt die Ergebnisse, wobei in dem Diagramm die optische Dichte o.D. in Abhängigkeit von der Pipettierungssequenz aufgetragen ist.

Bei dem für die Untersuchungen gewählten ELISA handelte es sich um einen Test zum Nachweis von IgG-Antikörpern gegen das Rötelnvirus. Der Testaufbau und die Funktionsweise sind in den Europäischen Patentanmeldungen 0 264 866 und 0 270 729 detailliert beschrieben worden.

Die in Figur 1 aufgetragenen Meßwerte der ausschließlich pipettierten Testkontrolle (P/N 296) zeigen, daß die Meßwerte zu Beginn der Serie höher als am Ende der Serie lagen. Die eingesetzte Testkontrolle besitzt einen Sollwert von 0,862. Es ist also zusätzlich ersichtlich, daß auch der letzte Wert der Serie noch zu hoch liegt.

Die Ursache für den beobachteten Effekt, d.h. für die beobachtete "Pipettierungsdrift", liegt darin, daß bereits beim normalerweise unter Zimmertemperatur durchgeführten Einpipettieren der Untersuchungsproben der nachzuweisende Analyt beginnt, an die feste Phase zu binden. Je länger diese ungewollte Vorlaufreaktion ist, desto stärker ist die Verfälschung des jeweiligen Meßwertes.

Die Beobachtung, daß auch noch der Meßwert der letzten Pipettierung über dem Sollwert liegt, beruht auf einer trendmäßigen Erhöhung aller Werte dieser Serie (Niveau).

Die Verfälschung der Meßwerte durch die "Pipettierungsdrift" führt immer zu einer mehr oder weniger ausgeprägten Erhöhung des Meßwertes einer Untersuchungsprobe in Abhängigkeit von der Position innerhalb der Serie.

Im Gegensatz zur "Pipettierungsdrift" ist der Tagestrend einer Serie (Niveau) definitionsgemäß für alle Untersuchungsproben gleich und kann die einzelnen Meßwerte sowohl nach oben als auch nach unten verfälschen.

Die Intensität der "Pipettierungsdrift" innerhalb einer Serie hängt von der Zimmertemperatur, der Temperatur der einpipettierten Proben, der Temperatur der Mikrotitrationsplatte und der Zeitdauer vom Einbringen der ersten bis zu letzten Untersuchungprobe ab.

Auch von Serie zu Serie verschlechtert die "Pipettierungsdrift" die Reproduzierbarkeit, weil sowohl die zuvor aufgeführten Randbedingungen wie Temperatur und Pipettierzeit, als auch die Probenpositionierung innerhalb der Serie von Tag zu Tag oder von Labor zu Labor unterschiedlich sein können.

Aufgabe der Erfindung ist es nun, ein Verfahren zu schaffen, mit dem die vorstehend beschriebene "Pipettierungsdrift" korrigiert werden kann, ohne daß die Wirksamkeit bestehender Korrekturverfahren eines für alle Untersuchungsproben gemeinsamen Trends ("Niveau-Korrektur") beeinflußt und insbesondere verringert wird.

Gelöst wird diese Aufgabe durch ein Verfahren, wie es im Anspruch 1 angegeben ist.

Weitere vorteilhafte Ausführungsformen der Erfindung sind in den Ansprüchen 2 bis 7 angegeben.

Zur Durchführung des Verfahrens gemäß der Erfindung wird zunächst einmal eine positive Testkontrolle der üblichen Art mit Angabe eines für den jeweiligen ELISA vorgeschriebenen Sollwertes benötigt. Diese Testkontrolle wird sowohl innerhalb einer Serie als auch in gleicher Weise von Serie zu Serie eingesetzt. Das Beschicken der Mikrotitrationsplatten beginnt jeweils mit einer Mehrfachbestimmung der positiven Testkontrolle, wird fortgesetzt mit dem Einpipettieren der Untersuchungsproben und endet wiederum mit einer Mehrfachbestimmung derselben positiven Testkontrolle. Als Mehrfachbestimmungen sind für die Zwecke dieser Erfindung meistens Doppelbestimmungen bereits ausreichend. Die Sequenz der einpipettierten Proben muß innerhalb der Geometrie der Mikrotitrationsplatte - z.B. horizontal oder vertikal - für das Verfahren der Erfindung festgelegt werden.

Nachdem der Test für eine Serie durchgeführt worden ist und die einzelnen Meßwerte vorliegen, werden die Mittelwerte (oder Mediane) der jeweiligen Mehrfachbestimmungen der positiven Testkontrolle am Anfang und am Ende der Serie ermittelt und miteinander verglichen, um das Ausmaß der Pipettierungsdrift zu erkennen. Wenn der Mittelwert der Mehrfachbestimmung am Anfang zahlenmäßig oder numerisch größer als der am Ende ist, wird das Vorliegen einer "Pipettierungsdrift" angenommen.

An dieser Stelle sei bemerkt, daß bei immunometrischen Tests, bei denen eine negative Korrelation zwischen der Konzentration des nachzuweisenden Analyts und der numerischen Meßwertgröße besteht, wie dies bei kompetitiven Tests der Fall ist, eine Pipettierungsdrift, d.h. ein systematischer Fehler, angenommen wird, wenn das Mittelmaß der Bestimmungen der Testkontrolle am Serienanfang kleiner als am Serienende ist.

Danach werden die beiden Mittelwerte der Testkontrolle am Anfang und am Ende in Relation zum Sollwert gesetzt. Bei einer "Pipettierungsdrift" weist der Mittelwert der Testkontrolle am Serienanfang eine bestimmte Abweichung zum Sollwert auf, der Mittelwert am Ende aber eine andere. Eine Meßwertkorrektur, beispielsweise über einen Korrekturfaktor, muß daher am Serienanfang anders als am Serienende sein. Gemäß der Erfindung wird nun die Meßwertkorrektur in Abhängigkeit von der Position einer Probe innerhalb der Pipettierungssequenz verändert. Zu diesem Zweck müssen Annahmen über den durch die Pipettierungsdrift hervorgerufenen systematischen Fehler gemacht werden.

In einer Ausführungsform der Erfindung wird eine Serie, beispielsweise vom Hersteller der Reagenzien, ausschließlich mit einer Testkontrolle durchgeführt, deren Sollwert bekannt ist. Der sich ergebende Kurvenverlauf aufgrund der Pipettierungsdrift wird dann als Kurvenverlauf des systematischen Fehlers für spätere Messungen angenommen. Das heißt, wenn bei Messungen von Untersuchungsproben die Mittelwerte am Anfang und am Ende der Serie gemäß der Erfindung ermittelt worden sind, wird angenommen, daß der Kurvenverlauf zwischen diesen Mittelwerten einem Kurvenverlauf gehorcht, wie er im Vorversuch an einer Testkontrolle gefunden worden ist. Mathematisch gesehen, wird eine theoretische Kurve durch diese real ermittelten Mittelwerte am Anfang und am Ende gelegt und die Meßwerte für die einzelnen Untersuchungsproben werden um einen Betrag vergrößert oder verkleinert, der entsprechend ihrer Position innerhalb der Pipettierungssequenz der Abweichung der angenommenen Korrekturkurve für die Testkontrollprobe vom Anfangs- oder Endwert der Testkontrollprobe an der derselben Position entsprechenden Stelle proportional ist.

Gemäß der Erfindung wird also die Korrektur der zwischen den Mehrfachbestimmungen der Testkontrolle innerhalb der Serie positionierten Untersuchungsproben in Abhängigkeit von ihrer Position innerhalb der Pipettierungssequenz unterschiedlich durchgeführt. Je weiter vorn eine Untersuchungsprobe in der Serie positioniert ist, desto ähnlicher wird der Korrekturfaktor dem der zu Anfang durchgeführten positiven Testkontrolle sein. Je weiter hinten in der Serie eine Untersuchungsprobe liegt, desto mehr ähnelt der Korrekturfaktor demjenigen der am Ende liegenden positiven Testkontrolle.

Mathematisch kann die in Pipettierrichtung der Untersuchungsproben zu verändernde Korrektur auf unterschiedliche Weise bestimmt werden. Eine einfache und in der Praxis im allgemeinen ausreichende Korrektur geht von der Annahme aus, daß der Kurvenverlauf des systematischen Fehlers zwischen Anfang und Ende der Serie linear ist. In diesem Falle können aus den Mittelwerten der positiven Testkontrolle die zugehörigen Korrekturfaktoren einfach berechnet werden, indem eine Gerade mit einer in Probeneingaberichtung negativen Steigung nach der Formel

$$y = m \cdot i + b$$

konstruiert wird. Wenn dann die optische Dichte o.D. der i-ten Probe als s(i), die gemittelte o.D. der Testkontrolle am Serienanfang als s(1) und die gemittelte o.D. der Testkontrolle am Serienende als s(n) bezeichnet werden, dann erhält man als Steigung m in vorstehender Formel

$$m = \frac{s(n) - s(1)}{n - 1}$$

und als Achsenabschnitt

$$b = s(1) \frac{s(n) - s(1)}{n - 1}$$

Daraus ergibt sich für jede Untersuchungsprobe ein Korrekturfaktor

$$k(i) = \frac{\text{Sollwert der Kontrolle}}{m \cdot i + b}$$

wobei i die Lage der Probe definiert.

Bei praktischen Anwendungen der Erfindung wird ein entsprechendes Rechenprogramm für einen Computer erstellt, der die Meßdaten der Mikrotitrationsplatte von einem Photometer übernimmt und automatisch korrigiert.

Im folgenden werden die Vorteile des erfindungsgemäßen Verfahrens anhand von Simulationsversuchen und Beispielen aus der Praxis demonstriert. Dabei wird auf die beigefügten Figuren und Tabellen Bezug genommen.

In den Zeichnungen zeigen:

Figur 1: Meßwerte der optischen Dichte o.D. in Abhängigkeit von der Position innerhalb der Pipettierungssequenz für eine Testkontrolle mit bekanntem Sollwert;

Figur 2: Meßwerte der optischen Dichte o.D. in Abhängigkeit von der Position innerhalb der Pipettierungssequenz an Untersuchungsproben in nicht korrigiertem und gemäß der Erfindung korrigiertem Verlauf;

Figur 3: eine Wiederholung der Serienmessung, die der Figur zugrundelag, durch den gleichen Testdurchführenden und

Figur 4: eine Wiederholung der Serienmessung, die der Figur zugrunde lag, durch einen anderen Testdurchführenden und

Figur 5: eine Wiederholung der Serienmessung, die der Figur zugrunde lag, durch den gleichen Testdurchführenden, der die Serienmessung von Figur 4 durchgeführt hat.

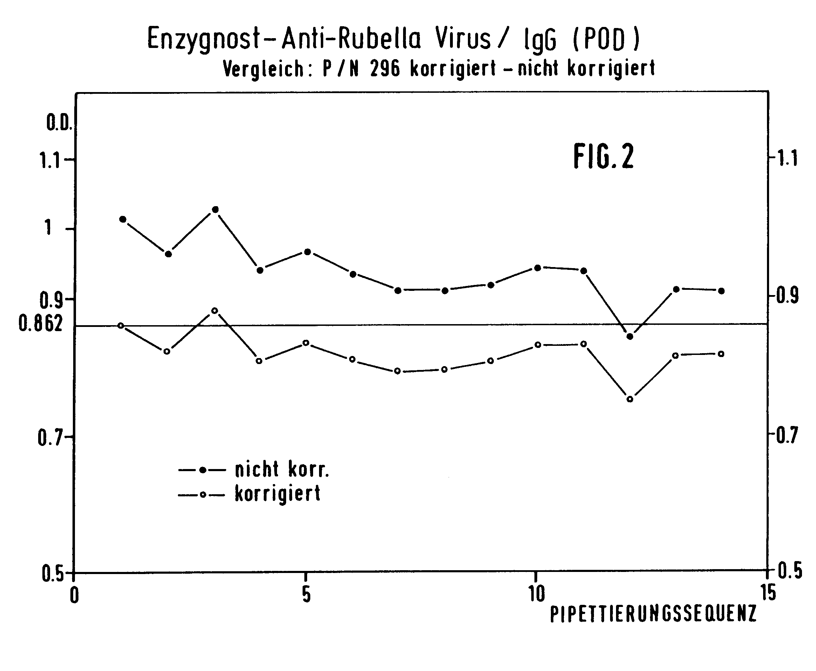

In der Tabelle 1 werden die Meßergebnisse der Figuren 2 bis 5 zahlenmäßig ausgewertet.

Figur 1 zeigt Meßwerte der optischen Dichte einer Testkontrolle, deren Sollwert bekannt ist, in dem bereits oben schon erwähnten ELISA-Test. Die Pipettierungsdrift ist deutlich erkennbar. Der Kurvenverlauf wurde als linear angenommen, wie die punktierte Kurve zeigt. Die gleiche Testkontrolle wurde gemäß der Erfindung als

TAB. 1

Enzygnost-Anti-Rubella Virus / IgG (POD)

Vergleich P/N 296 nicht korrigiert - korrigiert

| Daten aus Abbildung | Richtigkeit* (Sollwert 0,862) | | Reproduzierbarkeit** (VK) | |
|---|---|---|---|---|
| | vor Korrektur | nach Korrektur | vor Korrektur | nach Korrektur |
| 2 | 0,931 | 0,819 | 4,9 % | 3,7 % |
| 3 | 0,960 | 0,865 | 7,2 % | 4,2 % |
| 4 | 0,714 | 0,819 | 8,7 % | 5,2 % |
| 5 | 0,716 | 0,836 | 15,1 % | 8,1 % |

* Mittelwert von 14 Bestimmungen in Serie

**innerhalb der Serie

Doppelbestimmung am Anfang und am Ende jeweils einer genau eine Mikrotitrationsplatte füllenden Serie eingesetzt. Zur Untersuchung des erfindungsgemäßen Verfahrens in der Praxis wurde dieselbe Testkontrolle dann alternierend mit Untersuchungsproben von Patienten in der vorher festgewählten Sequenz (hier vertikal) einpipettiert. Die Patientenproben wurden für die Kurvendarstellungen der Abbildungen 2 bis 5 nicht berücksichtigt, sie dienten nur dazu, die Praxisbedingungen (Zeitaufwand, Wechsel der Pipettenspitzen usw.) zu simulieren.

In Figur 2 sind die für die innerhalb der Serie verteilt angeordneten Testkontrollen erzielten Meßwerte vor und nach der erfindungsgemäßen Korrektur dargestellt. Auf der x-Achse ist die Pipettierungssequenz, auf der y-Achse der Meßwert der optischen Dichte o.D. skaliert. Die bei einer o.D. von 0,862 eingetragene Horizontale repräsentiert den Sollwert der Testkontrolle. Das Ergebnis der Doppelbestimmung der Testkontrolle am Anfang und am Ende der Serie ist nicht eingetragen.

Bei der Kurve der nicht korrigierten Werte läßt sich durch Vergleich mit dem Sollwert leicht erkennen, daß alle Werte ein zu hohes Niveau haben. Gleichzeitig ist der systematische Fehler durch die Pipettierungsdrift als zusätzliche Werteverfälschung umso stärker ausgeprägt, je früher die Probe einpipettiert wurde. Die Meßwertkorrektur nach dem erfindungsgemäßen Verfahren verringert beide nachteiligen Effekte.

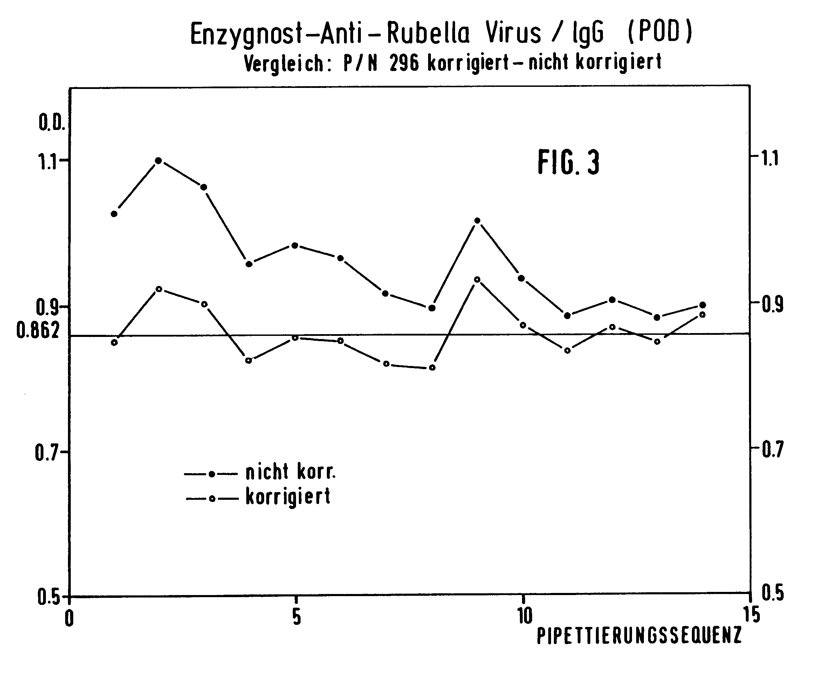

Dieselbe Person wiederholte den gesamten Ansatz, was zu einem ähnlichen Bild führte, wie Figur 3 zeigt. Lediglich die Pipettierungsdrift war etwas stärker ausgeprägt. Die Korrektur nach dem erfindungsgemäßen Verfahren lieferte eine wesentliche Verbesserung der Richtigkeit der Meßwerte.

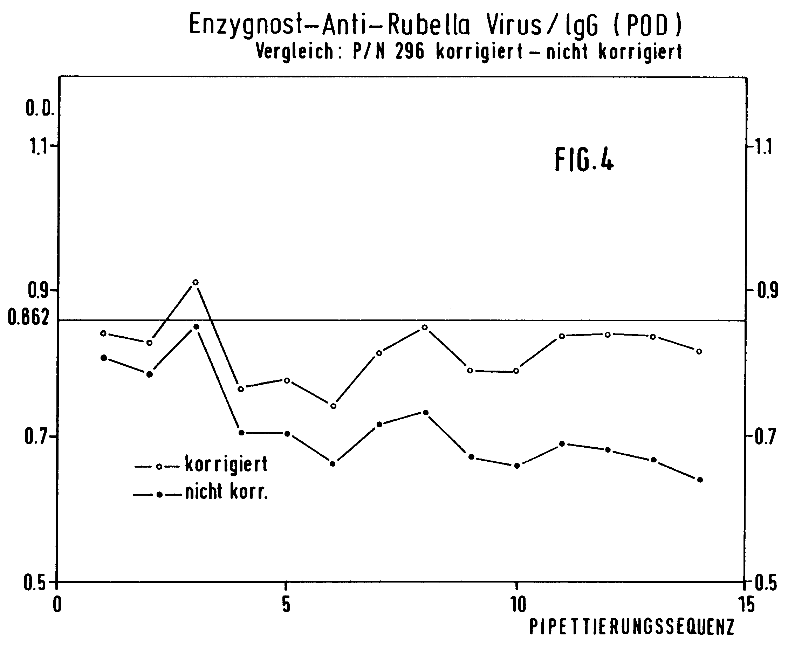

Bei einer anderen Person, die mit den gleichen Reagenzien genau denselben Testansatz fuhr, ergab sich ein anderer Kurvenverlauf, der in Figur 4 dargestellt ist. Hier lag das Niveau der nicht korrigierten Werte deutlich zu niedrig, gleichzeitig war aber eine Pipettierungsdrift zu erkennen. Die Korrektur nach dem erfindungsgemäßen Verfahren kompensierte die beiden nachteiligen Effekte.

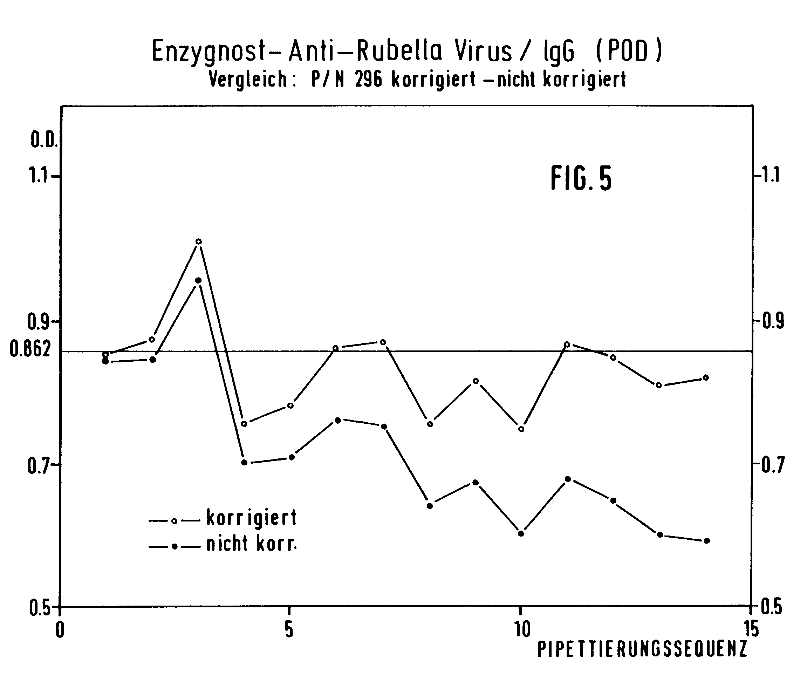

In der Wiederholung dieses Ansatzes ergab sich auch bei dieser gleichen Person ein ähnlicher Kurvenverlauf, wie Figur 5 zeigt. Wiederum verbesserte das erfindungsgemäße Verfahren die einzelnen Meßwerte in Bezug zum Sollwert von 0,862 ganz deutlich.

Bei den bisher geschilderten Versuchen simulierte die innerhalb der Serie verteilte Testkontrolle den Positionseffekt verschiedener Untersuchungsproben mit unbekanntem Gehalt an Antikörpern gegen das Rötelnvirus. Dies hat den Vorteil, nicht nur die Reproduzierbarkeit, sondern auch die Richtigkeit der Meßwerte vor und nach der erfindungsgemäßen Korrektur vergleichen zu können. Die entsprechenden Werte sind in Tabelle 1 aufgetragen.

Bemerkenswert ist, daß die erfindungsgemäße Korrektur unabhängig vom jeweiligen Niveau der Meßwerte arbeitet. Unabhängig davon, ob die Serie im Trend zu hoch (Figuren 2 und 3) oder zu niedrig (Figuren 4 und 5) lag, wurde die Pipettierungsdrift immer in der Weise korrigiert, daß sowohl Richtigkeit als auch Reproduzierbarkeit der Meßwerte verbessert wurden.

Aufbauend auf diesem Ergebnis wurden die experimentellen Ausgangsbedingungen für das erfindungsgemäße Verfahren in mehreren Punkten erschwert:

1) es wurden Patientenproben untersucht,

2) es wurde die Reproduzierbarkeit von Serie zu Serie geprüft, wobei die Positionierung der einzelnen Proben innerhalb der Serie wechselte, und

3) es wurde nicht nur mit dem oben angeführten ELISA zum Nachweis von virusspezifischem IgG sondern zusätzlich auch mit einem weiteren Test gearbeitet, der IgM gegen Rötelnvirus nachweist. Im letzteren Fall wurde die für diese Testvariante vorgeschriebene Testkontrolle eingesetzt, deren Sollwert bei einer o.D. von 0,392 lag.

Die Ergebnisse sind in Tabelle 2 aufgetragen und zeigen wiederum eindeutig, daß das erfindungsgemäße Verfahren auch die Meßwerte von Proben aus der Praxis bezüglich der Reproduzierbarkeit verbessert. Die Richtigkeit konnte nicht bewertet werden, weil diese Proben keinen Sollwert besitzen.

Da bei diesen Untersuchungen eine größere Datenmenge (insgesamt 9 Proben mit Serienlängen von n = 10 bis n = 14) verfügbar war, läßt sich die durch das erfindungsgemäße Verfahren erzielte Verbesserung der Reproduzierbarkeit von Serie zu Serie grob abschätzen. Durchschnittlich sinkt bei dem gewählten ELISA-Test der Variationskoeffizient VK von 13,7 % auf 9,4%.

TAB.2

VERGLEICH PATIENTENPROBEN NICHT KORRIGIERT - KORRIGIERT

ENZYGNOST-ANTI-RUBELLA VIRUS / IgG (POD)

| Probenidenti-fikation | Reproduzierbarkeit von Serie zu Serie | | | | | |
|---|---|---|---|---|---|---|
| | vor Korrektur | | | nach Korrektur | | |
| | n | x̄ | Vk | n | x̄ | Vk |
| 068810 | 12 | 1,267 | 13,5 % | 12 | 1,338 | 10,0% |
| 7/190 | 12 | 1,314 | 16,7 % | 12 | 1,390 | 7,5% |
| Pool VII | 12 | 2,333 | 8,3 % | 12 | 2,468 | 5,7% |

ENZYGNOST-ANTI-RUBELLA VIRUS / IgM (POD)

| Probenidentifikation | | n | x̄ | Vk | n | x̄ | Vk |
|---|---|---|---|---|---|---|---|
| 411818 | | 14 | 0,425 | 17,4 % | 14 | 0,400 | 11,3 % |
| 242 | | 14 | 0,384 | 17,0 % | 14 | 0,361 | 13,6 % |
| 249 | | 14 | 0,472 | 15,1 % | 14 | 0,428 | 9,5 % |
| 6528 | | 10 | 0,385 | 13,4 % | 10 | 0,337 | 9,5 % |
| 263 | | 14 | 1,051 | 11,4 % | 14 | 0,988 | 10,1 % |
| Pool VII | | 14 | 0,782 | 10,5 % | 14 | 0,735 | 7,8 % |

Abschätzen der Verbesserung:     $\sum$ 13,7 %                                      $\sum$ 9,4%

**Patentansprüche**

1.  Verfahren zur Verbesserung von Richtigkeit und Reproduzierbarkeit der Meßdaten immunometrischer Tests, die unter Verwendung von Mikrotitrationsplatten durchgeführt werden, dadurch gekennzeichnet, daß die Reihenfolge des Einbringens der zu untersuchenden Proben auf die Mikrotitrationsplatte für eine Serie festgelegt wird, und daß ein innerhalb einer Serie von Untersuchungsproben auftretender, systematischer Fehler ("Pipettierungsdrift"), der die Meßwerte der Untersuchungsproben in unterschiedlichem und nicht genau bestimmbarem Ausmaß verfälscht, korrigiert wird, indem eine Testkontrollprobe mit einem für den Test benannten Meßergebnis (Sollwert) als Mehrfachbestimmung zu Anfang der Serie, d.h. durch mindestens zweimaliges Einbringen als Probe vor Beginn des Einbringens der eigentlichen Untersuchungsproben der Serie, und am Ende der Serie nach dem Einbringen der eigentlichen Untersuchungsproben ebenfalls als Mehrfachbestimmung, d.h. durch Einbringen von mindestens zwei Proben der Testkontrollprobe, eingesetzt wird und aus der Veränderung des Mittelwertes der Meßwerte für die Testkontrollprobe am Ende im Vergleich zu dem Mittelwert am Anfang ein Korrekturfaktor für die einzelnen Untersuchungsproben der Serie abgeleitet wird.

2. Verfahren nach Anspruch 1, dadurch gekennzeichnet, daß zur Ableitung des Korrekturfaktors für die Untersuchungsproben in erster Näherung eine lineare Änderung des Mittelwertes der Meßwerte der Testkontrollprobe vom Anfang zum Ende der Serie in Abhängigkeit von der laufenden Probennummer i auf der Mikrotitrationsplatte angenommen wird und die zwischen den Meßwerten der Testkontrollprobe am Anfang und am Ende erhaltenen Meßwerte der Untersuchungsproben korrigiert werden, indem die in Pipettierrichtung liegende i-te Probe mit einem Korrekturfaktor k

$$k(i) = \frac{\text{Sollwert der Kontrolle}}{m \cdot i + b}$$

korrigiert wird, wobei

$$m = \frac{s(n) - s(1)}{n - 1}$$

die Steigung der angenommenen Verbindungsgeraden zwischen den Mittelwerten am Anfang und am Ende ist und

$$b = s(1) \quad \frac{s(n) - s(1)}{n - 1}$$

ist, wenn s(1) den gemittelten Anfangsmeßwert und s(n) den gemittelten Endmeßwert der Testkontrollprobe bedeuten.

3. Verfahren nach Anspruch 1, dadurch gekennzeichnet, daß zur Ableitung des Korrekturfaktors für die Untersuchungsproben ein durch experimentelle Versuche mit Testkontrollprobenserien ermittelter Kurvenverlauf für die Änderung des Mittelwertes der Meßwerte der Testkontrollprobe mit der laufenden Probennummer i auf der Mikrotitrationsplatte vom Anfang zum Ende der Serie angenommen wird und die Meßwerte für die einzelnen Untersuchungproben um einen Betrag vergrößert oder verkleinert werden, der entsprechend ihrer Position i der Abweichung der angenommenen Korrekturkurve für die Testkontrollprobe vom Anfangs- oder Endwert für die Testkontrollprobe an der der Position i entsprechenden Stelle proportional ist.

4. Verfahren nach einem der Ansprüche 1 bis 3, dadurch gekennzeichnet, daß bei immunometrischen Tests, bei denen eine positive Korrelation zwischen der Konzentration des nachzuweisenden Analyts und der numerischen Meßwertgröße besteht, ein systematischer Fehler ("Pipettierungsdrift") angenommen und korrigiert wird, wenn der Mittelwert der Meßwerte für die Testkontrollprobe am Serienanfang größer als am Serienende ist.

**5.** Verfahren nach einem der Ansprüche 1 bis 3, dadurch gekennzeichnet, daß bei immunometrischen Tests, bei denen eine negative Korrelation zwischen der Konzentration des nachzuweisenden Analyts und der numerischen Meßwertgröße besteht, wie beispielsweise bei kompetitiven Tests, ein systematischer Fehler ("Pipettierungsdrift") angenommen und korrigiert wird, wenn der Mittelwert der Meßwerte für die Testkontrollprobe am Serienanfang kleiner als am Serienende ist.

**6.** Verfahren nach einem der Ansprüche 2 bis 5, dadurch gekennzeichnet, daß der angenommene Kurvenverlauf der Änderung des Mittelwertes der Meßwerte der Testkontrollprobe als eine stufenweise Änderung angenommen wird, wobei die Anzahl der Stufen der Anzahl der Untersuchungsprobe entspricht.

**7.** Verfahren nach einem der Ansprüche 1 bis 6, dadurch gekennzeichnet, daß die erhaltenen bezüglich des sytematischen Fehlers durch die Pipettierdrift korrigierten Werte für die Untersuchungsproben in an sich bekannter Weise weiterhin einer Korrektur eines alle Untersuchungsproben gleichermaßen verfälschenden systematischen Fehlers ("Tagesniveau") unterworfen werden.

### Claims

**1.** A process for improving the accuracy and reproducibility of the data measured in immunometric tests carried out using microtitration plates, which comprises establishing the sequence in which the samples to be examined are introduced onto the microtitration plate for a series, and correcting a systematic error ("pipetting drift") which appears within a series of samples under examination and which falsifies the values measured on the samples under examination to a varying extent which cannot accurately be determined, by employing a test control sample having a measured result nominated for the test (standard value) as a multiple determination at the start of the series, i.e. by introducing it as a sample at least twice before starting the introduction of the actual samples under examination in the series, and by employing it similarly as a multiple determination at the end of the series after the introduction of the actual samples under examination, i.e. by introducing at least two samples of the test control sample, and deriving a correction factor for the individual samples under examination in the series from the change in the mean value of the values measured for the test control sample at the end, compared with the mean value at the start.

**2.** The process as claimed in claim 1, wherein the correction factor for the samples under examination is derived, to a first approximation, by assuming a linear variation in the mean value of the values measured on the test control sample from the start to the end of the series as a function of the serial sample number i on the microtitration plate, and correcting the values measured on the samples under examination obtained between the values measured on the test control sample at the start and at the end by correcting the ith sample in the direction of pipetting by a correction factor k

$$k(i) = \frac{\text{standard value of the control}}{m \cdot i + b}$$

where

$$m = \frac{s(n) - s(1)}{n - 1}$$

is the gradient of the connecting straight lines assumed between the mean values at the start and at the end and

$$b = s(1) \frac{s(n) - s(1)}{n - 1}$$

if s(l) denotes the averaged initial value and s(n) denotes the averaged final value measured on the test control sample.

3. The process as claimed in claim 1, wherein the correction factor for the samples under examination is derived by assuming, for the change in the mean value of the values measured on the test control sample as a function of the serial sample number i on the microtitration plate from the start to the end of the series, a shape of curve determined by experimental tests with series of test control samples, and increasing or reducing the values measured for the individual samples under examination by an amount which is proportional, depending on its position i, to the deviation of the assumed correction curve for the test control sample from the initial or final value for the test control sample at the point corresponding to the position i.

4. The process as claimed in any of claims 1 to 3, wherein a systematic error ("pipetting drift") is assumed in the case of immunometric tests in which there is a positive correlation between the concentration of the analyte to be determined and the numerical magnitude of the value measured, and this error is corrected if the mean value of the values measured for the test control sample is greater at the start of the series than at the end of the series.

5. The process as claimed in any of claims 1 to 3, wherein a systematic error ("pipetting drift") is assumed in the case of immunometric tests in which there is a negative correlation between the concentration of the analyte to be determined and the numerical magnitude of the value measured, such as, for example, in the case of competitive tests, and this error is corrected if the mean value of the values measured for the test control sample is smaller at the start of the series than at the end of the series.

6. The process as claimed in any of claims 2 to 5, wherein the assumed shape of the curve for the change in the mean value of the values measured on the test control sample is assumed to be a stepwise change, the number of steps corresponding to the number of the samples under investigation.

7. The process as claimed in any of claims 1 to 6, wherein the corrected values for the samples under investigation obtained in respect of the systematic error caused by pipetting drift are also subjected in a manner known per se to a correction for a systematic error ("day's level") which falsifies all the samples under examination to the same extent.

**Revendications**

1. Procédé pour améliorer l'exactitude et la reproductibilité des données de mesure de tests immunométriques, qui sont exécutés moyennant une utilisation de plaques de microtitrage, caractérisé en ce qu'on fixe la succession d'introduction des échantillons à examiner sur la plaque de microtitrage, pour une série, et qu'on corrige une erreur systématique ("dérive due au prélèvement à la pipette"), qui apparaît dans une série d'échantillons d'examen et qui altère les valeurs de mesure des échantillons d'examen à un degré variable, qui ne peut pas être déterminé de façon précise, par le fait qu'on utilise un échantillon de contrôle de test avec un résultat de mesure (valeur de consigne) désigné pour le test, en tant que détermination multiple au début de la série, c'est-à-dire à l'aide d'une insertion, exécutée au moins deux fois, en tant qu'échantillon avant le début de l'introducton des échantillons d'examen proprement dits de la série, et à la fin de la série après l'introduction des échantillons d'examen proprement dits, également en tant que détermination multiple, par exemple par introduction d'au moins deux échantillons de contrôle de test et contient un facteur de correction pour les différents échantillons d'examen de la série, à partir de la modification de la moyenne des valeurs de mesure pour l'échantillon de contrôle de test, obtenue à la fin, par rapport à la moyenne obtenue au départ.

2. Procédé selon la revendication 1, caractérisé en ce que pour l'obtention du facteur de correction pour les échantillons d'examen, on suppose en première approximation une variation linéaire de la moyenne des valeurs de mesure de l'échantillon de contrôle de test du début à la fin de la série en fonction du numéro courant i de l'échantillon sur la plaque de microtitrage, et on corrige les valeurs de mesure des échantillons d'examen, obtenues entre les valeurs de mesure de l'échantillon de contrôle de test au début et à la fin, en corrigeant le i-ème échantillon, situé dans la direction de prélèvement à la pipette, avec un facteur de correction k

$$k(i) = \frac{\text{valeur de consigne de l'échantillon de contrôle}}{m \cdot i + b}$$

avec

$$m = \frac{s(n) - s(1)}{n - 1}$$

représentant la pente de la droite supposée reliant les moyennes au départ et à la fin, et avec

$$b = s(1) - \frac{s(n) - s(1)}{n - 1}$$

s(1) désignant la valeur de mesure moyenne de départ et s(n) la valeur de mesure finale de l'échantillon de contrôle de test.

3. Procédé selon la revendication 1, caractérisé en ce que pour l'obtention du facteur de correction pour les échantillons d'examen, on suppose une allure de courbe déterminée par des essais expérimentaux avec des séries d'échantillons de contrôle de test pour la modification de la moyenne des valeurs de mesure de l'échantillon de contrôle de test portant le numéro courant i sur la plaque de microtitrage, du début à la fin de la série, et on augmente ou on réduit les valeurs de mesure pour les différents échantillons d'examen, d'une valeur qui, en fonction de la position i, est proportionnelle à l'écart de la courbe de correction supposée pour l'échantillon de contrôle de test à partir de la valeur de départ ou de la valeur finale pour l'échantillon de contrôle de test au niveau de l'emplacement correspondant à la position i.

4. Procédé selon l'une des revendications 1 à 3, caractérisé en ce que dans le cas de tests immunométriques, dans lesquels il existe une corrélation positive entre la concentration du composé à l'analysé devant être détecté et la grandeur numérique de la valeur de mesure, on suppose et on corrige une erreur systématique ("dérive due au prélèvement à la pipette") lorsque la moyenne des valeurs de mesure pour l'échantillon de contrôle de test est plus élevée au début de la série qu'à la fin de cette dernière.

5. Procédé selon l'une des revendications 1 à 3, caractérisé en ce que dans le cas de tests immunométriques, dans lesquels il existe une corrélation positive entre la concentration du composé à l'analysé devant être détecté et la grandeur numérique de la valeur de mesure, comme par exemple dans le cas de tests compétitifs, on suppose et on corrige une erreur systématique ("dérive due au prélèvement à la pipette"), lorsque la moyenne des valeurs de mesure pour l'échantillon de contrôle de test est plus faible au début de la série qu'à la fin de cette dernière.

**6.** Procédé suivant l'une des revendications 2 à 5, caractérisé en ce qu'on suppose que l'allure supposée de la courbe de la variation de la moyenne des valeurs de mesure de l'échantillon de contrôle de test est une variation échelonnée, le nombre des échelons correspondant au nombre de l'échantillon d'examen.

**7.** Procédé suivant l'une des revendications 1 à 6, caractérisé en ce que dans les valeurs obtenues, dont l'erreur systématique produite par la dérive due au prélèvement à la pipette sont corrigées, pour les échantillons d'examen, on corrige en outre de façon connue en soi une erreur systématique ("niveau journalier"), qui fausse de façon uniforme tous les échantillons d'examen.

FIG.1

Enzygnost–Anti-Rubella Virus / IgG (POD)

Vergleich: P / N 296 korrigiert – nicht korrigiert

FIG. 2

—•— nicht korr.
—○— korrigiert

PIPETTIERUNGSSEQUENZ

EP 0 374 686 B1

Enzygnost–Anti–Rubella Virus / IgG (POD)
Vergleich: P/N 296 korrigiert–nicht korrigiert

FIG. 3

nicht korr.
korrigiert

PIPETTIERUNGSSEQUENZ

**Enzygnost–Anti–Rubella Virus / IgG ( POD )**
Vergleich: P/N 296 korrigiert – nicht korrigiert

FIG.4

—o— korrigiert
—•— nicht korr.

PIPETTIERUNGSSEQUENZ

EP 0 374 686 B1

Enzygnost-Anti-Rubella Virus / IgG (POD)
Vergleich: P/N 296 korrigiert -nicht korrigiert

FIG. 5

—o— korrigiert
—•— nicht korr.

PIPETTIERUNGSSEQUENZ

EP 0 374 686 B1